# EUROPEAN PATENT SPECIFICATION

(11) **EP 1 715 863 B1**
(45) Date of publication and mention of the grant of the patent: **09.05.2012**
(21) Application number: 05712023.0
(22) Date of filing: 26.01.2005
(51) Int. Cl.: A61K 31/445, A61K 31/40, A61K 31/4525, A61K 45/06

(54) **ENHANCEMENT OF AMPAKINE-INDUCED FACILITATION OF SYNAPTIC RESPONSES BY CHOLINESTERASE INHIBITORS**
VERBESSERUNG DER DURCH AMPAKINE INDUZIERTEN ERLEICHTERUNG VON SYNAPTISCHEN REAKTIONEN DURCH CHOLINESTERASE-INHIBITOREN
RENFORCEMENT DE LA FACILITATION INDUITE PAR AMPAKINE DES REPONSES SYNAPTIQUES PAR LES INHIBITEURS DE LA CHOLINESTERASE

(30) Priority: 26.01.2004 US 539422 P
(43) Date of publication of application: 02.11.2006
(73) Proprietor: CORTEX PHARMACEUTICALS, INC., Irvine, CA 92618 (US); The Regents of The University of California, Oakland, CA 94607-5200 (US)
(72) Inventor: STAUBLI, Ursula, Laguna Beach, CA 92651 (US); LYNCH, Gary, Irvine, CA 92612 (US); ZHONG, Sheng, Aliso Viejo, CA 92656 (US); COLGIN, Laura, Mesa, AZ 85204-5804 (US)
(74) Representative: Baldock, Sharon Claire
(86) International application number: PCT/US2005/002372
(87) International publication number: WO 2005/072345

(56) References cited:
- EP-A- 1 203 584
- US-A- 5 747 492
- US-B2- 6 689 795
- JOHNSON STEVEN A ET AL: "Randomized, double-blind, placebo-controlled international clinical trial of the Ampakine CX516 in elderly participants with mild cognitive impairment. A progress report" JOURNAL OF MOLECULAR NEUROSCIENCE, BIRKHAEUSER, CAMBRIDGE, MA, US, vol. 19, no. 1/2, 1 January 2002 (2002-01-01), pages 197-200, XP008093759 ISSN: 0895-8696
- DANYSZ W: "CX-516 CORTEX PHARMACEUTICALS" CURRENT OPINION IN INVESTIGATIONAL DRUGS, PHARMAPRESS, US, vol. 3, no. 7, 1 January 2002 (2002-01-01), pages 1081-1088, XP008093723 ISSN: 1472-4472

## Description

### RELATED APPLICATIONS

This application claims the benefit of U.S. provisional patent application No. 60/539,422, filed January 26, 2004.

### BACKGROUND OF THE INVENTION

### Field of Invention

The present invention relates to the important discovery that acetylcholinesterase inhibitors, drugs that delay the breakdown of the transmitter acetylcholine and thereby increase the degree to which it stimulates acetylcholine receptors, potently amplify the effect of drugs that positively modulate AMPA-type (α-amino-3-hydroxy-5-methyl-isoxazole-4-propionic acid-type) glutamate receptors (ampakines and other receptor potentiators). Additional studies showed that other types of drugs that directly bind to and stimulate muscarinic-type acetylcholine receptors produce the same effect. Thus, increasing the receptor stimulating effect of released acetylcholine, or directly stimulating a class of receptors that recognizes released acetylcholine, amplifies the physiological effects of AMPA receptor potentiators. Relying upon this unexpected effect, this invention provides for a method of enhancing the physiological potency of AMPA receptor potentiators and a method of treating various psychiatric and neurodegenerative diseases in a patient by the co-administration of an AMPA receptor potentiator and an acetylcholinesterase inhibitor or subtype specific acetylcholine receptor agonist.

The release of glutamate at synapses at many sites in the mammalian brain stimulates two classes of postsynaptic receptors. These classes are usually referred to as AMPA/quisqualate and N-methyl-D-aspartic acid (NMDA) receptors. AMPA/quisqualate receptors mediate a voltage-independent fast excitatory post-synaptic current ("fast EPSC") whereas NMDA receptors generate a voltage-dependent, slow excitatory current. Studies carried out in slices of hippocampus or cortex indicate that AMPA receptors contribute to the dominant component of the excitatory post-synaptic current (EPSC) at most glutamatergic synapses under most circumstances (Honore et al., Science 241:701-703 (1988); Hestrin et al., Journal of Physiology (London) 422: 203-225 (1990); Stem et al., Journal of Physiology (London) 449:247-278 (1992)).

AMPA receptors are found in high concentrations in neocortex (see Petralia and Wenthold, in The Journal of Comparative Neurology 318:329-354 (1992)), in each of the major synaptic zones of hippocampus (see Baude et al., in Neuroscience 69:1031-1055 (1995)), and in the striatal complex (see Bernard et al., in The Journal of Neuroscience 17:819-833 (1997)). Studies in animals and humans indicate that these structures organize complex perceptual-motor processes and provide the substrates for higher-order behaviors (O'Keefe and Dostrovsky, Brain Research 34:171-175 (1971); McNaughton et al., Experimental Brain Research 52:41-49 (1983); Zola-Morgan et al., The Journal of Neuroscience 6:2950-2967 (1986); Knowlton et al., Science 273:1399-1402 (1996); Kitabatake et al., PNAS.100:7965-7970 (2003)). Thus, AMPA receptors mediate transmission in those brain networks responsible for a host of cognitive activities. It is therefore believed that drugs that enhance AMPA receptor mediated synaptic responses will enhance such activities, particularly under conditions in which they are reduced by disease or other conditions. On the other hand, AMPA receptors are also found in the spinal cord and brainstem. There is thus a possibility that drugs that enhance the receptors might disturb walking, respiration, heart rate, and therefore not be useful as therapeutic agents, especially if high concentrations are needed.

AMPA receptor mediated synaptic responses, if produced repetitively in rapid succession, can produce a depolarization that is of sufficient magnitude and duration to remove a voltage block that normally inactivates the above mentioned NMDA-type glutamate receptors. This event is followed by the induction of long-term potentiation (LTP), a form of synaptic modification widely regarded as the substrate of certain types of memory (Morris et al., Nature 319:774-776 (1986); McNaughton et al., The Journal of Neuroscience 6:563-571 (1986); Moser et al., Science 281:2038-2042; see Martin et al., Annual Review of Neuroscience 23:649:711 (2000) and Goosens and Maren, Hippocampus 12:592-599 (2002) for reviews). For this reason, it is believed that drugs capable of enhancing AMPA receptor mediated synaptic responses can facilitate the induction of LTP (as has been demonstrated, see Staubli et al., PNAS 91:11158-11162 (1994) for an example) and thus enhance memory.

AMPA receptor mediated synaptic responses, and the NMDA receptors they control, are known to regulate the expression of various genes, including those that produce trophic factors such as Brain-Derived Neurotrophic Factor (BDNF) and Nerve Growth Factor (NGF) (Zafra et al., Embo Jounal 9:3545-3550 (1990); Patterson et al., Neuron 9:1081-1088 (1992); Dragunow et al., Neuroscience Letters 160:232-236 (1993); Castren et al., Neuroreport 4:895-898 (1993); see Lindholm et al., JNeurobiol 25:1362-1372 (1994) and Leβmann, Gen Pharmac 31:667-674 (1998) for reviews). There is a large body of literature indicating that these trophic factors will be useful in treating neurodegenerative diseases such as Alzheimer's disease, Parkinson's disease, Huntington's disease, as well as the brain damage that results from stroke or head injury (see Sendtner et al., Nature 345:440-441 (1990); Kordower et al., PNAS 91:10898-10902 (1994); Hefti, Nature Medicine 3:497-498 (1997); Perez-Navarro et al., J Neurochem 75:2190-2199 (2000); Schabitz et al., Stroke 31:2212-2217 (2000); Bjarkam et al., Biogerontology 2:193-207 (2001); Philips et al., J Neurosurg 94:765-774 (2001) for examples). Thus, it is likely that drugs capable of enhancing AMPA receptor mediated synaptic responses will increase the production of brain trophic factors and thereby have utility in the treatment of the afore-mentioned brain degenerative conditions. As noted, however, there is also a clear probability that AMPA receptor potentiators, particularly at sufficiently high concentrations, would cause life-threatening disturbances of brain operations. Thus, there exists a need to devise a means of enhancing the therapeutic effects of AMPA receptor potentiators without increasing the dosages used in therapy.

Ampakines are the initial class of AMPA receptor potentiators that facilitate AMPA receptor mediated monosynaptic responses (EPSCs) in the brains of living animals after peripheral administration. The drugs are disclosed in International Patent Application Publication No. WO 94/02475 (PCT/US93/06916) (Lynch and Rogers, Regents of the University of California). The invention of ampakines made possible the testing of the potential therapeutic applications noted in the previous three paragraphs and as well determinations of whether such potential benefits could be obtained in the absence of serious side-effects. Preclinical experiments established that doses of peripherally administered ampakines that increase synaptic responses in cortex do not cause gross disturbances to brain operations (see Staubli et al., PNAS 91:777-781 (1994); Larson et al., The Journal of Neuroscience 15:8023-8030 (1995) for examples); nonetheless, seizures may be induced for most of the drugs at 10-20 times the concentrations needed to produce desired physiological or behavioral effects. These results were followed by the invention of several additional groups of structurally different forms of compounds that potentiate the operation of AMPA receptors; the arrival of these new families necessitates the use of the term 'AMPA potentiators' to indicate a broad group of compounds with similar effects on AMPA receptors (see, for examples, Quirk and Nisenbaum, CNS Drug Rev 8:255-282 (2002); O'Neill et al., Curr Drug Targets CNS Neurol Disord 3:181-194 (2004)). Ampakines and other AMPA potentiators have positive effects in animal models of various psychiatric disorders that involve cognitive disturbances. This work included models of attention deficit/hyperactivity disorder (Gainetdinov et al., PNAS 98:11047-11054 (2001)), schizophrenia (Larson et al., Brain Research 738:353-356 (1996); Johnson et al., J Pharmacol Exp Ther 289:392-397 (1999); Hess et al., Neuroscience 121:509-521 (2003)), depression (Li et al., Neuropharmacology 40:1028-1033 (2001); Knapp et al., European Journal of Pharmacology 440:27-35 (2002)), and anxiety (Zarate et al., Ann NY Acad Sci 1003:273-291 (2003)). The drugs were also found to promote both the induction of LTP and various types of memory (Staubli et al., PNAS 91:777-781 (1994); Staubli et al., PNAS 91:11158-11162 (1994); Larson et al., The Journal of Neuroscience 15:8023-8030 (1995); Rogan et al., The Journal of Neuroscience 17:5928-5935 (1997); Shors et al., Neuroscience Letters 186:153-156 (1995); Hampson et al., The Journal of Neuroscience 18:2740-2747 (1998)). Finally, AMPA potentiators increase the expression of trophic factors *in vitro* and *in vivo* (Lauterborn et al., The Journal of neuroscience 20:8-21 (2000); Legutko et al., Neuropharmacology 40:1019-1027 (2001); Mackowiak et al., Neuropharmacology 43:1-10 (2002); Lauterborn et al., The Journal of Pharmacology and Experimental Therapeutics 307:297-305 (2003)), and reduce brain damage in animal models of stroke and Parkinson's disease (Murray et al., J Pharmacol Exp Ther 306:752-762 (2003); Dicou et al., Brain Research 970:221-225 (2003); O'Neill et al., European Journal of Pharmacology 486:163-174 (2004)). Certain of these animal results have been confirmed in humans (Lynch et al., Exp Neurol 145:89-92 (1997); Ingvar et al., Exp Neurol 146:553-559 (1997); Goff et al., J Clin Psychopharmacol 21:484-487 (2001)); thus, ampakines are reported to have beneficial effects in treating brain disorders such as schizophrenia and age-related losses of memory, and the drugs are now being tested in multiple clinical trials . Johnson et al., Journal of Molecular Neuroscience 19:197-200 (2002) discloses a clinical trial of the Ampakine^{®} CX516 in elderly participants with mild cognitive impairment. US 5,747,492 A discloses heteroatom substituted benzoyl derivatives that enhance synaptic responses mediated by AMPA receptors.

Based on the above results, AMPA receptor potentiators are now regarded as a promising new class of pharmaceuticals with a very broad range of possible applications. It is of importance then to identify means for enhancing their potency. Minimally, such treatments would allow the production of a given level of synaptic facilitation using lower concentrations of AMPA receptor potentiators than would otherwise be required. Beyond this, there are AMPA receptor potentiators that have relatively small effects on synaptic responses but possess other very desirable properties, such as binding to a preferred subset of AMPA receptors, having a metabolic half-life appropriate to a particular use, altering the waveform of the AMPA receptor mediated synaptic current in a desired way, or being particularly free of side-effects. The present inventors have discovered an unexpected means for enhancing the physiological effects of AMPA receptor potentiators, including weak varieties of the type just noted, using compounds that are already approved for human use (i.e., acetylcholinesterase inhibitors).

It is generally accepted that the physiological role of acetylcholinesterase (AChE) is the rapid hydrolysis and inactivation of acetylcholine. Inhibitors of AChE enhance the effect of acetylcholine released from axon terminals throughout the peripheral and central nervous systems. There are many structural classes of AChE inhibitors. The pyrrolidinoindoline class is used therapeutically in the treatment of Alzheimer's dementia. See, Roszkowski, U.S. Pat. No. 4,647,580; Gutman et al. U.S. Pat. No. 6,492,522. In addition, phenserine and phenserine analogs are known acetylcholinesterase inhibitors and have been reported as useful in the treatment of Alzheimer's diseases. U.S. Pat. Nos. 5,306,825 and 5,734,062. Giacobini, Ed., "Current Res. In Alz. Therapy: Cholinesterase Inhibitors" p. 237-245, 1988 and Comfort, A. "Cholinersterase Inhibition In Treatment Of Alzheimer's Dementia", The Lancet, vol. 1, No. 5065, Mar. 25, 1978, p. 659-660 are two general review of this area.. US 6,689,795 B2 discloses methods for treating dementia by administering a therapeutically effective amount of at least one cholinesterase inhibitor compound, preferably donepezil hydrochloride or ARICEPT^{®} EP 1 203 584 A1 discloses a combined drug consisting of an acetyl-cholinesterase inhibitor associated with a cholinergic precursor for use in treating cognitive disorders.

AChE inhibitors are rarely used as cognitive enhancers outside of Alzheimer's disease. And even within that application, they do not have potent effects in that memory loss and dementia are still in evidence (Keltner et al., Perspect Psychiatr Care 37:31-34 (2001); Kaduszkiewicz et al., Fortschr Neurol Psychiatr 72:557-563 (2004); Tanaka et al., J Neurol Sci 225:135-141 (2004)). Moreover, they are believed to be effective in only a subgroup of patients in the earlier stages of the disease (*i.e*., mild to moderate Alzheimer's disease).

There are numerous drugs that directly stimulate acetylcholine receptors in the brain. Pilocarpine and carbachol are most commonly used to activate muscarinic-type acetylcholine receptors; nicotine is frequently used to activate the nicotinic class of acetylcholine receptors. There are multiple subclasses of both muscarinic-type and nicotinic-type acetylcholine receptors. Physostigmine will enhance the effects of released acetylcholine on both classes of receptors. Muscarinic drugs are shown by the inventors to amplify the effects of amapkines; this indicates that the enhanced effects of acetylcholine at such receptors accounts for at least part of the enhancing effect of AChE inhibitors. However, it remains possible that some part of the effect may be due in part to one of the several categories of nicotinic receptors. While drugs that enhance muscarinic-type acetylcholine receptors amplify the effects of ampakines in brain slice experiments, their potential utility for this purpose *in vivo* is greatly limited by their pronounced and undesirable effects on the body. Therefore, the present inventors will throughout this application describe the combination treatments as involving AChE inhibitors (which are currently employed for treating brain diseases) and AMPA receptor potentiators. It should be noted that the material taught herein indicates that if a drug were to be developed that stimulates muscarinic-type acetylcholine receptors with minimal effects outside the brain, then such a compound could be used to amplify the physiological and therapeutic effects of AMPA receptor potentiators.

The observation that AChE inhibitors, as well as drugs that stimulate muscarinic-type acetylcholine receptors, amplify the effects of AMPA receptor potentiators on monosynaptic responses generated by the transmitter glutamate constitutes a surprising discovery. Acetylcholine is not usually released at synapses using glutamate (the ligand for AMPA receptors) as a transmitter, and thus there is no reason to think that enhancing acetylcholine-mediated transmission or stimulating acetylcholine receptors would increase the effects of ampakines or other AMPA receptor potentiators. This novel and surprising discovery opens the way to a combination therapy in which one drug (AChE inhibitor), which, though safe, currently has a very limited application, is used to magnify the effects of a second drug class that appears to be useful across a broad spectrum of psychiatric illnesses (see above for examples).

### BRIEF SUMMARY OF THE INVENTION

Generally, the invention relates to a technique for enhancing the potency of ampakines and other AMPA receptor potentiators in living animals, so as to enhance the therapeutic value of such drugs. Increased potency allows for the use of lower dosages of the drugs and for the use of AMPA receptor potentiators having desirable pharmacological properties but relatively weak effects on AMPA receptor mediated synaptic responses. In one aspect, the present invention provides a method for treating a cognitive disorder in an animal. The method comprises the step of administering an effective amount of an AMPA receptor potentiator and an effective amount of an acetylcholinesterase inhibitor to the animal. In some embodiments, the potentiator is an ampakine, for example, CX717, which is from the benzofurazan carboxamide family of Ampakine potentiators (see below). In other embodiments, the acetylcholinesterase inhibitor is donepezil hydrochloride. In some embodiments, the cognitive disorder to be treated is Alzheimer's Disease, senile dementia, Attention Deficit Disorder (ADD), mild cognitive impairment (MCI), schizophrenia, depression, sexual dysfunction, anxiety, or impaired performance after sleep deprivation. In other embodiments, the animal being treated is a human. In some cases, the administration of the AMPA receptor potentiator and the acetylcholinesterase inhibitor is by peripheral administration.

In another aspect, the invention describes a means for treating degenerative disorders or brain injuries that are responsive to brain trophic factors by the co-administration of an effective amount of an AMPA receptor potentiator and an effective amount of a acetylcholinesterase inhibitor to a patient. In some embodiments, the degenerative disorder to be treated is Alzheimer's disease, Parkinson's disease, Huntington's disease, or Mild Cognitive Impairment. In some embodiments, the brain injury to be treated is caused by stroke or head trauma. In some embodiments, the patient is a human patient.

In another aspect, the present invention provides for a method for enhancing the therapeutic effect of an AMPA receptor potentiator. This method comprises the step of coadministering an effective amount of the potentiator and an effective amount of an acetylcholinesterase inhibitor to a patient. In some embodiments, the potentiator is an ampakine. An exemplary potentiator is CX717, a member of the benzofurazan carboxamide family of AMPA receptor potentiators. In other embodiments, the acetylcholinesterase inhibitor is donepezil hydrochloride. In some cases, the administration of the potentiator and acetylcholinesterase inhibitor is by peripheral administration.

In yet another aspect, the present invention provides for a composition, which comprises an effective amount of an AMPA receptor potentiator, an effective amount of an acetylcholinesterase inhibitor, and a physiologically acceptable carrier. In some embodiments, the potentiator is an AMPAkine, such as CX717. In some embodiments, the acetylcholinesterase inhibitor is donepezil hydrochloride. In some cases, the composition is formulated for peripheral administration, *e.g*., oral administration.

### BRIEF DESCRIPTION OF THE DRAWINGS

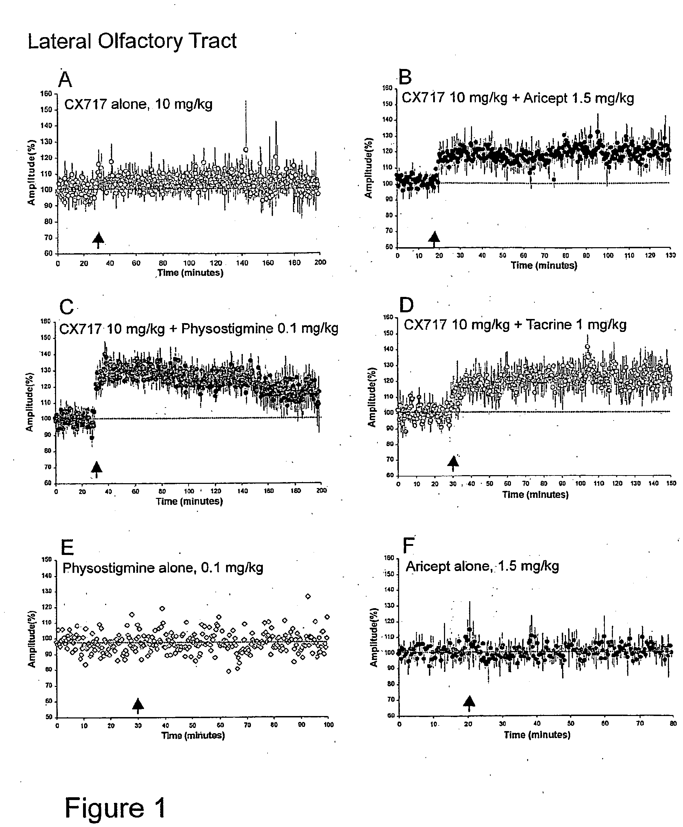

**Figure 1**: Effects of the ampakine CX717 at a dosage of 10 mg/kg on lateral olfactory tract (LOT) evoked responses in awake rats were enhanced by co-administration of various acetylcholinesterase inhibitors. In each panel, the time of intraperitoneal injection of the drug(s) is indicated by an arrow. Response amplitude measures were normalized to average pre-drug baseline values. **(A)** CX717 administered by itself at a dosage of 10 mg/kg produced very small (<10%) increases in LOT response amplitudes. **(B)** When CX717 (10 mg/kg) was co-injected with the acetylcholinestase inhibitor Aricept (1.5 mg/kg), LOT responses increased by approximately 20% above baseline, an effect that was approximately two-fold greater than that produced by CX717 alone. **(C)** Co-application of CX717 (10 mg/kg) and the acetylcholinesterase inhibitor physostigmine (0.1 mg/kg) produced increases in LOT response amplitude that were approximately three-fold greater than those obtained with CX717 alone. **(D)** Co-injection of CX717 (10 mg/kg) and the acetylcholinesterase inhibitor tacrine (1 mg/kg) produced increases in LOT response size that were more than 20% above pre-drug baseline values and more than two times greater than effects produced by the ampakine alone. **(E)** Physostigmine injected on its own at a dosage of 0.1 mg/kg did not detectably affect LOT responses. **(F)** When administered alone, Aricept (1.5 mg/kg) produced little to no effect on LOT responses.

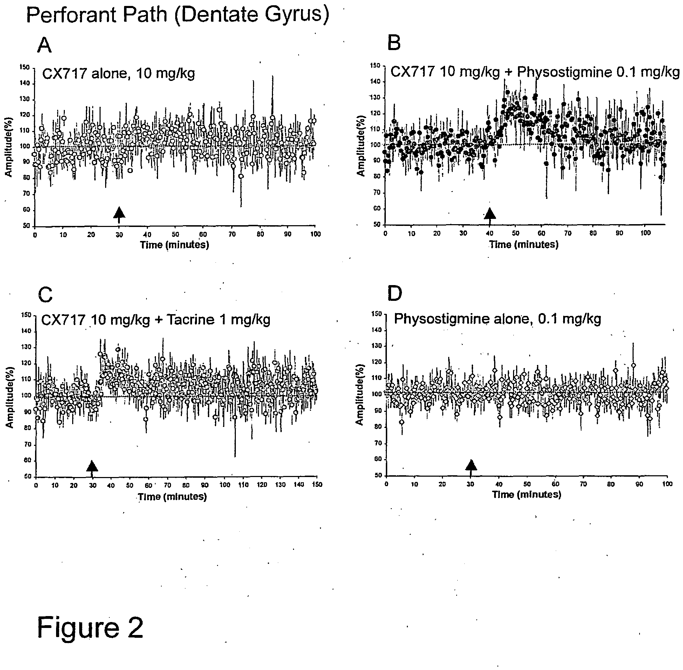

**Figure 2****:** Effects of the ampakine CX717 (10 mg/kg) on perforant path evoked responses in the dentate gyrus of awake rats were enhanced by co-administration of acetylcholinesterase inhibitors. In each panel, the time of intraperitoneal injection of the drug(s) is indicated by an arrow. Response amplitude measures were normalized to average pre-drug baseline values. **(A)** When administered alone at a dosage of 10 mg/kg, CX717 did little to affect perforant path response size. **(B)** Co-injection of CX717 (10 mg/kg) and the acetylcholinesterase inhibitor physostigmine (0.1 mg/kg) produced increases in perforant path response amplitude that were at least 20% greater than pre-drug baseline response amplitudes and significantly higher than the effects of CX717 alone. **(C)** When co-administered with the acetylcholinesterase inhibitor tacrine (1 mg/kg), CX717 (10 mg/kg) increased the size of perforant path responses by approximately 13% above baseline. **(D)** Injection of physostigmine alone at a concentration of 0.1 mg/kg did not detectably affect perforant path responses.

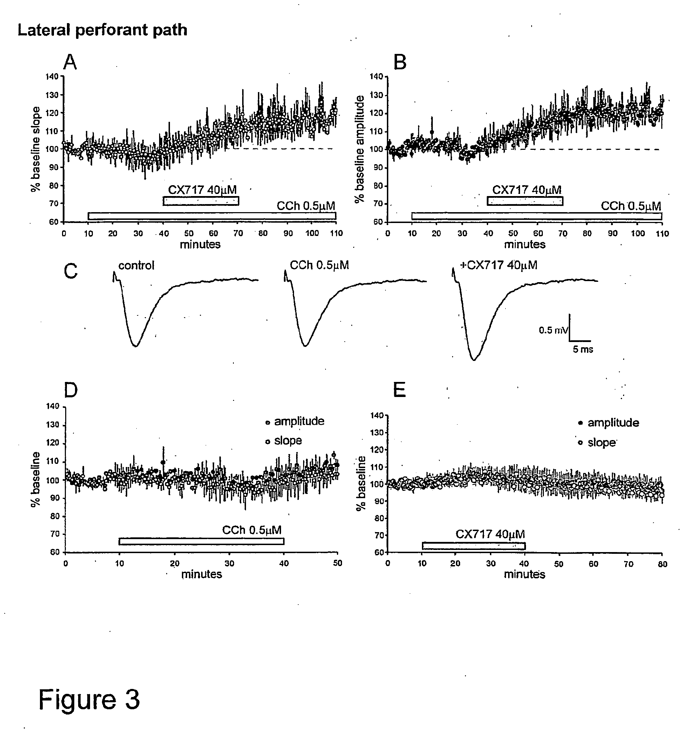

**Figure 3****:** Effects of the ampakine CX717 on lateral perforant path EPSPs in hippocampal slices were enhanced by co-application of the cholinergic agonist, carbachol (CCh). Measures depicted were normalized to pre-drug baselines. **(A** and **B)** CCh was infused at a concentration of 0.5 µM for a total period of 100 minutes. At 30 minutes after the start of CCh treatment, CX717 was infused at a concentration of 40 µM for a period of 30 minutes and produced significant increases in both slope (A) and amplitude (B). **(C)** Representative evoked responses are shown during baseline (left trace), during CCh application just prior to the start of CX717 infusion (middle trace), and at the end of CX717 infusion (right trace). **(D)** 0.5 µM CCh by itself did not affect the amplitude or slope of evoked responses. **(E)** 40 µM CX717 by itself only slightly increased the slope and amplitude of lateral perforant path EPSPs.

### DEFINITIONS

The term "alkyl," by itself or as part of another substituent, means, unless otherwise stated, a straight or branched chain, or cyclic hydrocarbon radical, or combination thereof, which may be fully saturated, mono- or polyunsaturated and can include di- and multivalent radicals, having the number of carbon atoms designated (*i.e*. C₁-C₁₀ means one to ten carbons). "Lower alkyl" refers to "alkyl" containing 1-4 carbon atoms. Examples of saturated hydrocarbon radicals include, but are not limited to, groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. Alkyl groups which are limited to hydrocarbon groups are termed "homoalkyl".

The term "alkylene" by itself or as part of another substituent means a divalent radical derived from an alkane, as exemplified, but not limited, by -CH₂CH₂CH₂CH₂-, and further includes those groups described below as "heteroalkylene." Typically, an alkyl (or alkylene) group will have from 1 to 24 carbon atoms, with those groups having 10 or fewer carbon atoms being preferred in the present invention. A "lower alkyl" or "lower alkylene" is a shorter chain alkyl or alkylene group, generally having eight or fewer carbon atoms.

The terms "alkoxy," "alkylamino" and "alkylthio" (or thioalkoxy) are used in their conventional sense, and refer to those alkyl groups attached to the remainder of the molecule via an oxygen atom, an amino group, or a sulfur atom, respectively.

The term "heteroalkyl," by itself or in combination with another term, means, unless otherwise stated, a stable straight or branched chain, or cyclic hydrocarbon radical, or combinations thereof, consisting of the stated number of carbon atoms and at least one heteroatom selected from the group consisting of O, N, P, Si and S, and wherein the nitrogen and sulfur atoms may optionally be oxidized and the nitrogen heteroatom may optionally be quaternized. The heteroatom(s) O, N, P and S and Si may be placed at any interior position of the heteroalkyl group or at the position at which the alkyl group is attached to the remainder of the molecule. Examples include, but are not limited to, -CH₂-CH₂-O-CH₃, - CH₂-CH₂-NH-CH₃, -CH₂-CH₂-N(CH₃)-CH₃, -CH₂-S-CH₂-CH₃, -CH₂-CH₂,-S(O)-CH₃, -CH₂-CH₂-S(O)₂-CH₃, -CH=CH-O-CH₃, -Si(CH₃)₃, -CH₂-CH=N-OCH₃, -CH=CH-N(CH₃)-CH₃, O-CH₃, -O-CH₂-CH₃, and-CN. Up to two heteroatoms may be consecutive, such as, for example, -CH₂-NH-OCH₃ and -CH₂-O-Si(CH₃)₃. Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini (*e.g*., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula -C(O)₂R'- represents both -C(O)₂R'- and -R'C(O)₂-. As described above, heteroalkyl groups, as used herein, include those groups that are attached to the remainder of the molecule through a heteroatom, such as -C(O)R', -C(O)NR', -NR'R", -OR', -SR, and/or - SO₂R'. Where "heteroalkyl" is recited, followed by recitations of specific heteroalkyl groups, such as -NR'R" or the like, it will be understood that the terms heteroalkyl and -NR'R" are not redundant or mutually exclusive. Rather, the specific heteroalkyl groups are recited to add clarity. Thus, the term "heteroalkyl" should not be interpreted herein as excluding specific heteroalkyl groups, such as -NR'R" or the like.

The terms "cycloalkyl" and "heterocycloalkyl", by themselves or in combination with other terms, represent, unless otherwise stated, cyclic versions of "alkyl" and "heteroalkyl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like.

The terms "halo" or "halogen," by themselves or as part of another substituent, mean, unless otherwise stated, a fluorine, chlorine, bromine, or iodine atom. Additionally, terms such as "haloalkyl," are meant to include monohaloalkyl and polyhaloalkyl. For example, the term "halo(C₁-C₄)alkyl" is meant to include, but not be limited to, trifluoromethyl, 2,2,2-trifluoroethyl, 4-chlorobutyl, 3-bromopropyl, and the like.

The term "aryl" means, unless otherwise stated, a polyunsaturated, aromatic, hydrocarbon substituent which can be a single ring or multiple rings (preferably from 1 to 3 rings) which are fused together or linked covalently. The term "heteroaryl" refers to aryl groups (or rings) that contain from one to four heteroatoms selected from N, O, and S, wherein the nitrogen and sulfur atoms are optionally oxidized, and the nitrogen atom(s) are optionally quaternized. A heteroaryl group can be attached to the remainder of the molecule through a heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems are selected from the group of acceptable substituents described below.

For brevity, the term "aryl" when used in combination with other terms (*e.g*., aryloxy, arylthioxy, arylalkyl) includes both aryl and heteroaryl rings as defined above. Thus, the term "arylalkyl" is meant to include those radicals in which an aryl group is attached to an alkyl group (*e.g*., benzyl, phenethyl, pyridylmethyl and the like) including those alkyl groups in which a carbon atom (*e.g*., a methylene group) has been replaced by, for example, an oxygen atom (*e.g*., phenoxymethyl, 2-pyridyloxymethyl, 3-(1-naphthyloxy)propyl, and the like).

The term "oxo" as used herein means an oxygen that is double bonded to a carbon atom.

Each of the above terms (*e.g*., "alkyl," "heteroalkyl," "aryl" and "heteroaryl") are meant to include both substituted and unsubstituted forms of the indicated radical. Preferred substituents for each type of radical are provided below.

Substituents for the alkyl, and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) can be one or more of a variety of groups selected from, but not limited to: -OR', =O, NR', N-OR', -NR'R", -SR', -halogen,-SiR'R"R"', -OC(O)R', -C(O)R', -CO₂R', -CONR'R", -OC(O)NR'R", -NR"C(O)R', -NR'-C(O)NR"R"', -NR"C(O)₂R', -NR-C(NR'R"R"')=NR"", -NR-C(NR'R")=NR'", -S(O)R', - S(O)₂R', -S(O)₂NR'R", -NRSO₂R', -CN and -NO₂ in a number ranging from zero to (2m'+1), where m' is the total number of carbon atoms in such radical. R', R", R'" and R"" each preferably independently refer to hydrogen, substituted or unsubstituted heteroalkyl, substituted or unsubstituted aryl, e.g., aryl substituted with 1-3 halogens, substituted or unsubstituted alkyl, alkoxy or thioalkoxy groups, or arylalkyl groups. When a compound of the invention includes more than one R group, for example, each of the R groups is independently selected as are each R', R", R'" and R"" groups when more than one of these groups is present. When R' and R" are attached to the same nitrogen atom, they can be combined with the nitrogen atom to form a 5-, 6-, or 7-membered ring. For example, -NR'R" is meant to include, but not be limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl (*e.g*., -CF₃ and -CH₂CF₃) and acyl (*e.g*., -C(O)CH₃, -C(O)CF₃, -C(O)CH₂OCH₃, and the like).

Similar to the substituents described for the alkyl radical, substituents for the aryl and heteroaryl groups are varied and are selected from, for example: halogen, -OR', =O, NR', =N-OR', -NR'R", -SR', -halogen, -SiR'R"R"', -OC(O)R', -C(O)R', -CO₂R', -CONR'R", -OC(O)NR'R", -NR"C(O)R', -NR'-C(O)NR"R'", -NR"C(O)₂R', -NR-C(NR'R"R'")=NR"", -NR-C(NR'R")=NR"', -S(O)R', -S(O)₂R', -S(O)₂NR'R", -NRSO₂R', -CN and -NO₂, -R', -N₃, - CH(Ph)₂, fluoro(C₁-C₄)alkoxy, and fluoro(C₁-C₄)alkyl, in a number ranging from zero to the total number of open valences on the aromatic ring system; and where R', R", R"' and R"" are preferably independently selected from hydrogen, alkyl, heteroalkyl, aryl and heteroaryl. When a compound of the invention includes more than one R group, for example, each of the R groups is independently selected as are each R', R", R'" and R"" groups when more than one of these groups is present.

Two of the substituents on adjacent atoms of the aryl or heteroaryl ring may optionally be replaced with a substituent of the formula -T-C(O)-(CRR')_{q}-U-, wherein T and U are independently -NR-, -O-, -CRR'- or a single bond, and q is an integer of from 0 to 3. Alternatively, two of the substituents on adjacent atoms of the aryl or heteroaryl ring may optionally be replaced with a substituent of the formula -A-(CH₂)ᵣ-B-, wherein A and B are independently -CRR'-, -O-, -NR-, -S-, -S(O)-, -S(O)₂-, -S(O)₂NR'- or a single bond, and r is an integer of from 1 to 4. One of the single bonds of the new ring so formed may optionally be replaced with a double bond. Alternatively, two of the substituents on adjacent atoms of the aryl or heteroaryl ring may optionally be replaced with a substituent of the formula - (CRR')ₛ-X'-(C"R"')_{d}-, where s and d are independently integers of from 0 to 3, and X' is -O-, - NR'-, -S-, -S(O)-, -S(O)₂-, or -S(O)₂NR'-. The substituents R, R', R" and R"' are preferably independently selected from hydrogen or substituted or unsubstituted (C₁-C₆)alkyl.

As used herein, the term "heteroatom" is meant to include oxygen (O), nitrogen (N), sulfur (S), phosphorus (P), and silicon (Si):

The term "pharmaceutically acceptable salts" is meant to include salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (*see,* for example, Berge et al., "Pharmaceutical Salts", Journal of Pharmaceutical Science, 1977, 66, 1-19). Certain specific compounds of the present invention contain both basic and acidic functionalities that allow the compounds to be converted into either base or acid addition salts.

The neutral forms of the compounds are preferably regenerated by contacting the salt with a base or acid and isolating the parent compound in the conventional manner. The parent form of the compound differs from the various salt forms in certain physical properties, such as solubility in polar solvents.

In addition to salt forms, the present invention provides compounds, which are in a prodrug form. Prodrugs of the compounds described herein are those compounds that readily undergo chemical changes under physiological conditions to provide the compounds of the present invention. Additionally, prodrugs can be converted to the compounds of the present invention by chemical or biochemical methods in an *ex vivo* environment. For example, prodrugs can be slowly converted to the compounds of the present invention when placed in a transdermal patch reservoir with a suitable enzyme or chemical reagent.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centers) or double bonds; the racemates, diastereomers, geometric isomers and individual isomers are encompassed within the scope of the present invention.

The compounds of the present invention may also contain unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds of the present invention, whether radioactive or not, are encompassed within the scope of the present invention.

Where two substituents are "optionally joined together to form a ring," the two substituents are covalently bonded together with the atom or atoms to which the two substituents are joined to form a substituted or unsubstituted aryl, a substituted or unsubstituted heteroaryl, a substituted or unsubstituted cycloalkyl, or a substituted or unsubstituted heterocycloalkyl ring.

The term "physiological effect of an AMPA receptor potentiator," as used herein, refers to the facilitation of synaptic responses induced by the release of glutamate from an axon terminal, or any physiological and biochemical consequences of such facilitation.

The term "therapeutic effect of an AMPA receptor potentiator," as used herein, refers to any detectable effect in preventing, reversing, inhibiting, or otherwise alleviating at least one symptom of any defined disease or condition that this AMPA receptor potentiator may be used in treatment therefor. For example, the therapeutic effect of an AMPA receptor potentiator may relate to the treatment of diseases and conditions such as Alzheimer's Disease, Parkinson's disease, Huntington's disease, senile dementia, Attention Deficit Disorder (ADD), mild cognitive impairment (MCI), schizophrenia, depression, sexual dysfunction, anxiety, impaired performance after sleep deprivation, or brain injury caused by stroke or head trauma.

The term "effective amount," as used herein, refers to an amount that produces therapeutic effects for which a substance is administered. The effects include the prevention, correction, or inhibition of progression of the symptoms of a disease/condition and related complications to any detectable extent. The exact amount will depend on the purpose of the treatment, and will be ascertainable by one skilled in the art using known techniques (*see, e.g*., Lieberman, Pharmaceutical Dosage Forms (vols. 1-3, 1992); Lloyd, The Art, Science and Technology of Pharmaceutical Compounding (1999); and Pickar, Dosage Calculations (1999)).

### DETAILED DESCRIPTION OF THE INVENTION

The present inventors have revealed for the first time that acetylcholinesterase inhibitors, and drugs that stimulate muscarinic-type acetylcholine receptors, markedly increase the effects of AMPA receptor potentiators on monosynaptic excitatory post-synaptic currents (EPSCs) generated through the release of the transmitter glutamate, binding of glutamate to AMPA-type glutamate receptors, and the opening of the AMPA receptor's channel. Based on this discovery, the invention provides for a method of enhancing the efficacy of AMPA receptor potentiators *in vivo* so that a given dosage of the drugs will elicit a larger physiological response or permit the production of a given physiological response with a lower concentration of the drugs.

### I. Diseases to Be Treated with the Present Invention

A variety of cognition related diseases may be treated with the method of the present invention. Exemplary disease states include attention deficit disorder (ADD), mild cognitive impairment (MCI), dementia, cognitive impairment in depressed patients, cognitive deterioration in individuals with Down's syndrome, neurodegeneration (*e.g*., Alzheimer's disease and Parkinson's disease), and schizophrenia. AMPA receptor potentiators enhance the production by brain cells of neurotrophic substances such as Brain-Derived Neurotrophic Factor (BDNF), and knowledge of the mechanisms whereby this effect is achieved indicates that the invention can also be used to enhance the production of such trophic materials above the level that could be achieved with an AMPA receptor potentiator alone. Accordingly, the invention can be used in the treatment of those degenerative disorders that respond to administration of BDNF and other trophic factors (see "Background of the Invention" section, above, for examples). The methods of treatment include administering to a patient in need of such treatment an effective amount of an AMPA receptor potentiator and an effective amount of an acetylcholinesterase inhibitor.

### II. AMPA Receptor Potentiators

Included in the methods, compositions and formulations of the present invention are AMPA receptor potentiators. AMPA receptor potentiators of the present invention are compounds or complexes of compounds that (1) bind to AMPA-type glutamate receptors, (2) cause that receptor to increase the ionic current it passes upon the binding of glutamate released from a pre-synaptic axon terminal, and (3) thereby increase the size of monosynaptic excitatory responses.

AMPA (α-amino-3-hydroxy-5-methyl-isoxazole-4-propionic acid) receptors are transmembrane glutamate binding proteins present in cells, particularly neurons. The binding of glutamate to an AMPA receptor normally gives rise to an influx of current into the target cell, which then causes the cell to generate a biological response. The biological response may be the generation of an action potential, changes in cellular secretion or metabolism, or induction of gene expression.

A wide variety of AMPA receptor potentiators are useful in the present invention, including ampakines (disclosed in International Patent Application Publication No. WO 94/02475 (PCT/US93/06916) (Lynch and Rogers, Regents of the University of California), U.S. Pat Nos. 5,773,434, 6,274,600, and 6,166,008, which are assigned to the same assignee as the present application); LY404187, LY 392098, LY503430, and derivatives thereof (produced by Eli Lilly, Inc.); CX546 and derivatives thereof; CX614 and derivatives thereof; S 18986-1 and derivatives thereof; benzoxazine AMPA receptor potentiators and derivatives thereof (as disclosed in U.S. Patent Nos. 5,736,543, 5,962,447, 5,773,434 and 5,985,871); heteroatom substituted benzoyl AMPA receptor potentiators and derivatives thereof (as disclosed in U.S. Patent Nos. 5,891,876, 5,747,492, and 5,852,008); benzoyl piperidines/pyrrolidines AMPA receptor potentiators and derivatives thereof as (disclosed in U.S. Patent No. 5,650,409); benzofurazan carboxamide AMPA receptor potentiators and derivatives thereof (as disclosed in U.S. Patent Nos. 6,110,935, 6,313,115 and 6,730,677); and 7-chloro-3-methyl-3-4-dihydro-2H-1,2,4 benzothiadiazine S,S, dioxide and derivatives thereof, as described in Zivkovic et al., 1995, J. Pharmacol. Exp. Therap., 272:300-309; Thompson et al., 1995, Proc. Nat. Acad. Sci. USA, 92:7667-7671.

AMPA receptor potentiators primarily act, not by directly stimulating AMPA receptors, but by "allosteric modulation" of the response of AMPA receptors to the natural ligand glutamate (i.e., a change in the conformation of the 3-dimensional receptor) and thereby increase the size and duration of synaptic transmission at synapses containing AMPA receptors. These compounds bind to the AMPA receptor at a site other than the glutamate binding site and such binding does not by itself give rise to ion fluxes. However, when a glutamate molecule binds to a glutamate receptor that has bound to it an AMPA receptor potentiator, the subsequent ion flux is of greater magnitude and duration. Thus, in the presence of the AMPA receptor potentiators used herein, postsynaptic neurons will have a larger response to a given transmitter (glutamate) release event than postsynaptic neurons that do not contain the AMPA receptor potentiators.

Compounds useful in the practice of this invention are generally those that amplify (upmodulate) monosynaptic excitatory responses by positively modulating AMPA-type glutamate receptors. We describe herein a wide variety of diverse compounds suitable for use in the invention.

In an exemplary embodiment, the AMPA receptor potentiator is a specific AMPA receptor potentiator. A specific AMPA receptor potentiator acts upon AMPA receptors by causing an increase in currents mediated by brain AMPA receptors expressed in Xenopus oocytes without affecting responses by γ-amino-butyric acid (GABA), kainic acid (KA), or NMDA receptors. In another exemplary embodiment, infusion of an AMPA receptor potentiator into slices of hippocampus substantially increases the size of fast synaptic potentials without altering resting membrane properties. In yet another exemplary embodiment, an AMPA receptor potentiator enhances synaptic responses at several sites in hippocampus and has no effects on NMDA-receptor mediated potentials. See, for example, Staubli et al., in Psychobiology 18:377-381 (1990) and Xiao et al., in Hippocampus 1:373-380 (1991). In another exemplary embodiment, the AMPA receptor potentiator has a rapid onset and washout and can be applied repeatedly with no apparent lasting effects. In a further exemplary embodiment, the AMPA receptor potentiator is administered peripherally by the oral route, or by subcutaneous injection, or by intravenous injection, or by intraperitoneal injection, and then enters the brain and facilitates AMPA receptor mediated synaptic responses recorded in the hippocampus.

In an exemplary embodiment, the AMPA receptor potentiator has the formula:

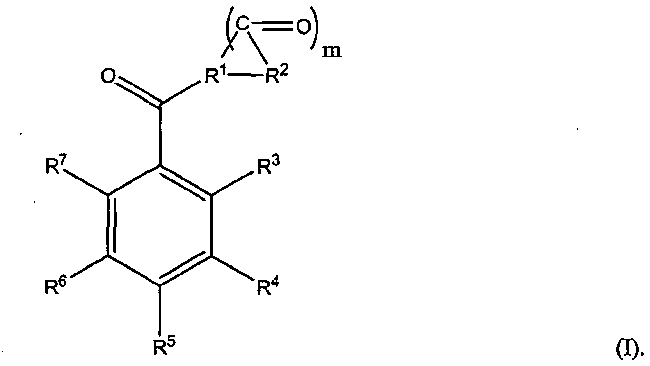

In Formula (I), R¹ is selected from N and CH. R² is selected from substituted or unsubstituted alkyl, and substituted or unsubstituted heteroalkyl.

R³ is selected from hydrogen, substituted or unsubstituted alkyl, and substituted or unsubstituted heteroalkyl. R³ is optionally joined with R² to form a substituted or unsubstituted fused ring substituent.

R⁴ is selected from hydrogen, -OH, substituted or unsubstituted alkyl, and substituted or unsubstituted heteroalkyl. R⁴ is optionally joined with R⁵ to form a substituted or unsubstituted fused ring substituent.

R⁵ is selected from hydrogen, -OH, substituted or unsubstituted alkyl, substituted or unsubstituted heteroalkyl, substituted or unsubstituted cycloalkyl, substituted or unsubstituted heterocycloakyl, substituted or unsubstituted aryl, and substituted or unsubstituted heteroaryl.

R⁶ is selected from hydrogen, substituted or unsubstituted alkyl, and substituted or unsubstituted heteroalkyl.

R⁷ is selected from hydrogen, substituted or unsubstituted alkyl, and substituted or unsubstituted heteroalkyl. R⁷ is optionally joined with R² to form a substituted or unsubstituted fused ring substituent.

In a related embodiment, the AMPA receptor potentiator has the formula:

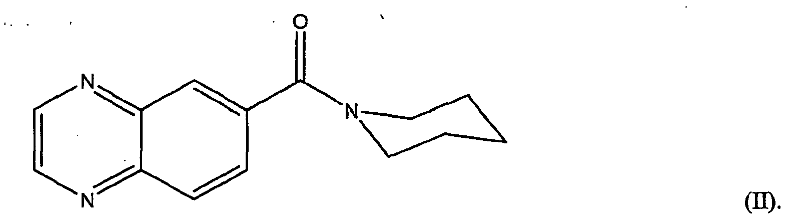

In another exemplary embodiment, the AMPA receptor potentiator has the formula:

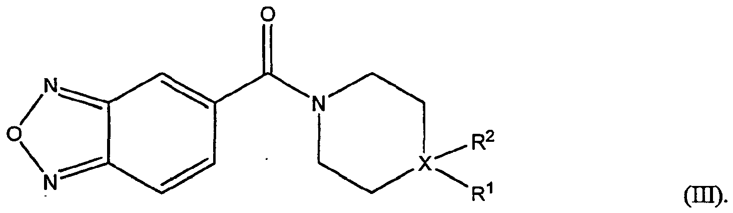

In Formula (III), X is selected from C, N, S and O. Where X is C, then R1 and R2 are independently selected from hydrogen, fluorine, -OH, and CN. Where X is N, then R1 is hydrogen or lower alkyl and R2 is absent. Where X is S, R1 and R2 are independently absent, or oxygen. Where X is O, R¹ and R2 are absent.

The compounds described by the formulae above additionally include pharmaceutically acceptable salts thereof.

### Generic Assays for Identifying AMPA Receptor Potentiators

Additional AMPA receptor potentiators may be identified using routine methods known to those skilled in the art. These methods involve a variety of accepted tests to determine whether a given candidate compound is an upmodulator of the AMPA receptor. The primary assay is measurement of enlargement of the excitatory postsynaptic potential (EPSP) in *in vitro* brain slices, such as rat hippocampal brain slices.

In experiments of this kind, slices of hippocampus from a mammal such as a rat are prepared and maintained in an interface chamber using conventional methods. For example, field EPSPs are recorded in the stratum radiatum of region CAlb and elicited by single stimulation pulses delivered once per 20 seconds to a bipolar electrode positioned in the Schaffer-commissural projections (see Granger et al., 1993, Synapse, 15:326-329; Staubli et al., 1994a, Proc. Nat. Acad. Sci., 91:777-781; Staubli et al., 1994b, Proc. Nat. Acad. Sci., 91:11158-11162).

The waveform of a normal EPSP is composed of: (a) an AMPA receptor component, which has a relatively rapid rise time in the depolarizing direction and which decays within about 20 msec; (b) an NMDA receptor component which has slow rise and decay times ; the NMDA portion is very small in normal media, because the NMDA receptor channel is blocked at resting membrane potential; (c) a GABA component in the opposite (hyperpolarizing) direction as the glutamatergic (AMPA and NMDA) components, exhibiting a time course with a rise time of about 10-20 msec and very slow decay (about 50-100 msec or more.

The different components can be separately measured to assay the effect of a putative AMPA receptor enhancing agent. This is accomplished by adding agents that block the unwanted components, so that the remaining, detectable responses are mediated by a single class of transmitter receptors (*i.e*., AMPA receptors only, or NMDA receptors only, or GABA receptors only). For example, to measure AMPA responses, an NMDA receptor blocker (*e.g*., AP-5 or other NMDA blockers known in the art) and/or a GABA blocker (*e.g*., picrotoxin or other GABA blockers known in the art) are added to the slice.

AMPA receptor potentiators useful in the present invention are substances that cause an increased ion flux through the AMPA receptor complex channels in response to release of glutamate. Increased ion flux is typically measured as one or more of the following non-limiting parameters: at least a 10% increase in the initial slope, amplitude, decay time, or the area under the curve of the post-synaptic response elicited by stimulation of presynaptic axons and recorded at synapses known to use glutamate as a transmitter. The response can be measured with intracellular recording (whole cell clamp method or sharp electrode method) from the post-synaptic neuron on which the stimulated synapses are formed or by extracellular recording using electrodes placed in proximity to the stimulated synapses. The post-synaptic response can be measured as current influx into the post-synaptic neuron (referred to as the Excitatory Post-Synaptic Current or `EPSP') or as a change in the membrane voltage of the post-synaptic neuron (referred to as the Excitatory Post-Synaptic Potential or 'EPSP') or as a field potential generated by the activated synapses (referred to as the field EPSP). These measurements can be readily collected in brain slices, typically taken from the hippocampus of a rat, treated to block NMDA and GABA receptors.

An additional and more detailed assay is that of excised patches, *i.e*., membrane patches excised from cultured hippocampal slices; methods are described in Arai et al., Brain Research 638:343-346 (1994). Outside-out patches are obtained from pyramidal hippocampal neurons and transferred to a recording chamber. Glutamate pulses are applied in order to elicit excitatory currents, and data are collected with a patch clamp amplifier and digitized (Arai *et al.,* 1994, *supra* and Arai et al., Neuroscience 25:573-585 (1996)).

Although these membrane patches should contain only glutamatergic receptors, any GABAergic currents or NMDA currents can be blocked as above (*e.g*., with picrotoxin and AP-5).

The preferred AMPA receptor potentiators to be used in the present invention are capable of entering the brain and possess the potency and metabolic stability needed to increase synaptic responses in living animals. The central action of a drug can be verified by measurement of monosynaptic field EPSPs in behaving animals (see Staubli *et al.,* 1994a, *supra*) and time course of biodistribution can be ascertained via injection and PET measurement of appropriately radiolabeled (C-11 or F-18) drug (see Staubli *et al.,* 1994b, *supra*).

### III. Acetylcholinesterase (AChE) Inhibitors

Included in the methods, compositions, and formulations of the present invention are acetylcholinesterase inhibitors. Acetylcholinesterase inhibitors of the present invention are compounds or complexes of compounds that are capable of inhibiting the action of the acetylcholinesterase enzyme. In an exemplary embodiment, the acetylcholinesterase inhibitor is capable of inhibiting the normal metabolic breakdown of acetylcholine by inhibiting the action of the acetylcholinesterase enzyme.

A wide variety of acetylcholinesterase inhibitors are useful in the present invention, including reversible acetylcholinesterase inhibitors and irreversible acetylcholinesterase inhibitors. Exemplary acetylcholinesterase inhibitors include, for example, neostigmine (prostigmin); physostigmine (antilirium); edrophonium (tensilon); soman; parathion, malathion, isoflurophate (floropryl); diisopropylflurorphosphate (DFP); echothiophate (phospholine); donepezil; galantamine; metrifonate; rivastigmine; tacrine; velnicrine; galatamine hydrobromide; 5,7-dihydro-3-[2-[1-(phenyl-methyl)-4-piperidinyl]ethyl]-6H-pyrrolo [3,2-f]-1,2-benzisoxazol-6-one, also called icopezil (See J. Med. Chem., 1995, 38, 2802-2808), MDL-73,745 or zifrosilone (See Eur. J. Pharmacol., 1995, 276, 93-99) and TAK-147 (J. Med. Chem., 1994, 37 2292-2299); those decribed in Drugs, 1997, 53(5), 752-768; The Merck Index, 12th edition; and derivatives thereof. Other examples of acetylcholinesterase inhibitors are those described in patent applications JP 09-095483, WO 97/13754, WO 97/21681, WO 97/19929, ZA 96-04565, U.S. Pat. No. 5,455,245, WO 95-21822, EP 637 586, U.S. Pat. No. 5,401,749, EP 742 207, U.S. Pat. No. 5,547,960, WO 96/20176, WO 96/02524, EP 677 516, JP 07-188177, JP 07-133274, EP 649 846, EP 648 771, JP 07-048370, U.S. Pat. No. 5,391,553, WO 94/29272 and EP 627 400.

One of the inhibitors is donepezil hydrochloride (ARICEPT^{™}). A reversible inhibitor of acetylcholinesterase, donepezil hydrochloride is known chemically as (±)-2,3-dihydro-5,6-dimethoxy-2-[[1-(phenylmethyl)-4-piperidinyl] methyl]-1H-inden-1-one hydrochloride. Donepezil hydrochloride is commonly referred to in the pharmacological literature as E2020. It has an empirical formula of C₂₄H₂₉NO₃ HCl and a molecular weight of 415.96. Donepezil hydrochloride is a white crystalline powder and is freely soluble in chloroform, soluble in water and in glacial acetic acid, slightly soluble in ethanol and in acetonitrile, and practically insoluble in ethyl acetate and in n-hexane.

ARICEPT^{™} is available for oral administration in film-coated tablets containing 5 or 10 mg of donepezil hydrochloride. Inactive ingredients are lactose monohydrate, cornstarch, microcrystalline cellulose, hydroxypropyl cellulose, and magnesium stearate. The film coating contains talc, polyethylene glycol, hydroxypropyl methylcellulose, and titanium dioxide. Additionally, the 10 mg tablet contains yellow iron oxide (synthetic) as a coloring agent.

Other acetylcholinesterase inhibitors useful in the present invention may be easily identified using a variety of methods well known in the art. For example, the Ellman assay for acetylcholinesterase inhibition (See G. L. Ellman, K. D. Courtney, V. Andrews, Jr., and R. M. Featherstone, Biochem. Pharmacol., vol. 7, 88-95 (196 1)) may be employed using the p-chloromethyl derivative to determine whether a given compound is capable of inhibiting the action of acetylcholinesterase. An additional useful assay was described in C. R. Mantione et al., J. Neurochem., 41, 251 (1983) using [¹⁴C]acetyleholine (1.9 mCi/mmol). Alternatively, a radioactive assay using [³H]-acetylcholine iodide as substrate may be implemented (Johnson, C. and Russel, R.L. Analytical Chemistry (1975) 64: 229-238). The assay of Johnson and Russel may also be modified as described by M. R Emmerling and H. M. Sobkowicz in Hearings Research, 32:137-146 (1988). See also M. J. Marks, D. M. Patinkin, L. D. Artman, J. B. Busch, and A. C. Collins in Pharmacol. Biochem. and Behav.15:271-279 (1981).

### IV. Formulations

The acetylcholinesterase inhibitor and the AMPA receptor potentiator compounds of this invention are incorporated into a variety of formulations for therapeutic administration. Examples are capsules, tablets, syrups, suppositories, and various injectable forms. Administration of the compounds is achieved in various ways, including oral, bucal, rectal, parenteral, intraperitoneal, intradermal, transdermal, etc., administration. Preferred formulations of the compounds are oral preparations, particularly capsules or tablets.

The compounds of the present invention are preferably formulated prior to administration. Therefore, another aspect of the present invention is a pharmaceutical formulation of the acetylcholinesterase inhibitor and the AMPA receptor potentiator compounds described above and a pharmaceutically-acceptable carrier, diluent, or excipient. The present pharmaceutical formulations are prepared by known procedures using well-known and readily available ingredients. In making the compositions of the present invention, the active ingredient(s) will usually be mixed with a carrier, or diluted by a carrier, or enclosed within a carrier, and may be in the form of a capsule, sachet, paper, or other container. When the carrier serves as a diluent, it may be a solid, semi-solid, or liquid material which acts as a vehicle, excipient, or medium for the active ingredient. The compositions can be in the form of tablets, pills, powders, lozenges, sachets, cachets, elixirs, suspensions, emulsions, solutions, syrups, aerosols, ointments containing, for example, up to 10% by weight of active compound, soft and hard gelatin capsules, suppositories, sterile injectable solutions, and sterile packaged powders.

Some examples of suitable carriers, excipients, and diluents include lactose, dextrose, sucrose, sorbitol, mannitol, starches, gum, acacia, calcium phosphate, alginates, tragacanth, gelatin, calcium silicate, microcrystalline cellulose, polyvinylpyrrolidone, cellulose, water syrup, methyl cellulose, methyl and propyl hydroxybenzoates, talc, magnesium stearate, and mineral oil. The formulations can additionally include lubricating agents, wetting agents, emulsifying and suspending agents, preserving agents, sweetening agents, or flavoring agents. Compositions of the invention may be formulated so as to provide quick, sustained, or delayed release of the active ingredient after administration to the patient by employing procedures well known in the art.

### V. Pharmaceutical Compositions and Administration

### A. Mode of Delivery

The co-administration of an AMPA receptor potentiator and an acetylcholinesterase inhibitor can be used for treating various cognitive or mental disorders that may be treated by enhancement of glutamatergic transmission. The AMPA receptor potentiator and the acetyl-cholinesterase inhibitor may be administered to a patient separately or in the same pharmaceutical composition. Thus, the present invention also provides a composition that comprises an effective amount of an AMPA receptor potentiator, *e.g*., an AMPAkine compound such as CX717, and an effective amount of an acetylcholinesterase inhibitor, *e.g*., donepezil hydrochloride.

Pharmaceutical compositions of the invention are suitable for use in a variety of drug delivery systems. Suitable formulations for use in the present invention are found in Remington's Pharmaceutical Sciences, Mack Publishing Company, Philadelphia, PA, 17th ed. (1985). For a brief review of methods for drug delivery, *see,* Langer, Science 249: 1527-1533 (1990).

The pharmaceutical compositions are intended for peritoneal, intranasal, topical, oral, or local administration, such as by subcutaneous injection, aerosol inhalation, or transdermal adsorption, for prophylactic and/or therapeutic treatment. Commonly, the pharmaceutical compositions are administered parenterally, *e.g*., subcutaneously or intravenously. Routes of administration may also be characterized as either central administration, *i.e*., direct delivery to the brain, or peripheral administration, *i.e*., indirect delivery to the brain, such as through oral administration, injection into the circulatory system, and the like, where the AMPA receptor potentiator and the acetylcholinesterase inhibitor must cross the blood-brain barrier to enter a patient's brain. Thus, the present invention provides pharmaceutical compositions for parenteral administration, which comprise an AMPA receptor potentiator and an inhibitor of acetylcholinesterase dissolved or suspended in a physiologically acceptable carrier, preferably an aqueous carrier, *e.g*., water, buffered water, saline, PBS, and the like. The compositions may contain pharmaceutically acceptable auxiliary substances as required to approximate physiological conditions, such as pH adjusting and buffering agents, tonicity adjusting agents, wetting agents, detergents, and the like.

These compositions may be sterilized by conventional sterilization techniques, or may be sterile filtered. The resulting aqueous solutions may be packaged for use as is, or lyophilized, the lyophilized preparation being combined with a sterile aqueous carrier prior to administration. The pH of the preparations typically will be between 3 and 11, more preferably from 5 to 9, and most preferably from 7 and 8.

The compositions containing the potentiator and inhibitor can be administered for prophylactic and/or therapeutic treatments. In therapeutic applications, compositions are administered to a patient already suffering from a disease or condition related to cognitive deficiencies, in an amount sufficient to cure or at least partially arrest the symptoms of the disease and its complications. An amount adequate to accomplish this is defined as a "therapeutically effective dose." Amounts effective for this use will depend on the severity of the disease or condition and the weight and general state of the patient.

In prophylactic applications, compositions containing the AMPA potentiator and acetylcholinesterase inhibitor are administered to a patient susceptible to or otherwise at risk of a cognitive disorder (*e.g*., Alzheimer's disease). Such an amount is defined to be a "prophylactically effective dose." In this use, the precise amounts again depend on the patient's state of health and weight.

Single or multiple administrations of the compositions can be carried out with dose levels and pattern being selected by the treating physician. In any event, the pharmaceutical formulations should provide a quantity of an AMPA receptor potentiator and an AChE inhibitor sufficient to effectively treat the patient for a cognitive disorder or reduce the patient's risk of developing the disorder.

### B. Dosage

The above described compounds and/or compositions are administered at a dosage that diminishes the symptoms of cognition disorders (see above) in subjects suffering from these disorders, while at the same time minimizing any side-effects. It is contemplated that the composition will be obtained and used under the guidance of a physician.

Typical dosages for systemic AMPA receptor potentiators administration range from about 0.01 to about 10 milligrams per kg weight of subject per administration. A typical dosage may be one 5-200 mg tablet taken once a day, or one time-release capsule or tablet taken once a day and containing a proportionally higher content of active ingredient. The time-release effect may be obtained by capsule materials that dissolve at different pH values, by capsules that release slowly by osmotic pressure, or by any other known means of controlled release.

Dose levels can vary as a function of the specific compound, the severity of the symptoms, and the susceptibility of the subject to side effects. Some of the specific compounds that stimulate glutamatergic receptors are more potent than others. Preferred dosages for a given compound are readily determinable by those of skill in the art by a variety of means. A preferred means is to measure the physiological potency of a given compound that is a candidate for administration, by the method of Davis et al. (1997), Psychopharmacology 133:161-167. Briefly, excised patches and excitatory synaptic responses are measured in the presence of different concentrations of test compounds, and the differences in dosage response potency are recorded and compared. Davis *et al.* found that one specific compound designated BDP-20 was about ten-fold more potent than another designated BDP-12 in a variety of behavioral (exploratory activity, speed of performance) and electrophysiological (excised patches and excitatory synaptic responses) tests. The relative physiological potency was an accurate measure of their behavioral potency. Thus, excised patches and excitatory synaptic responses may be used to gauge the relative physiological (and behavioral) potency of a given compound with regard to a known standard.

AMPA receptor potentiator compounds for the treatment of brain disorders may have a half-life measured from less than 10 minutes to more than 6 hours. In some embodiments, the compound preferably has a rapid onset and short elimination half-life. In yet another embodiment, the compound has a rapid onset and a 4- to 24-hour elimination half-life.

In the present invention, the AMPA receptor potentiators are typically administered together with AChE inhibiting compounds. Although the inhibitors are effective in their normal therapeutic range, compounds are preferably administered close to or at their optimal therapeutic doses. The range of therapeutically effective doses for mammalian subjects ranges from about 0.02 to about 0.2 mg per kilogram of body weight per day, or preferably between about 0.1 mg/kg to about 0.5 mg/kg of body weight per day, more preferably between about 10 mg/kg to about 250 mg/kg, depending on the particular AChE inhibitor administered, route of administration, dosage schedule and form, and general and specific responses to the drug.

There are at least four well-known acetylcholinesterase inhibitors that have been commercially marketed. They are: 1) **tacrine hydrochloride,** commercially known as Cognex and prescribed in single dosages of 10 to 50 mg each used four times daily; 2) **donepezil hydrochloride,** commercially known as Aricept and prescribed in single dosages of 5 to 10 mg daily; 3) **rivastigmine tartrate,** commercially known as Exelon and prescribed in dosages from 1.5 to 6 mg that are administered twice daily; and 4) **galantamine hydrobromide,** commercially known as Reminyl and prescribed in dosages from 4 to 16 mg that are administered twice daily. Additionally, known or related compounds include neostigmine (Prostigmin), physostigmine (Antilirium), edrophonium (Tensilon), and metrifonate.

For convenience, the total daily dosage may be divided and administered in portions throughout the day, if desired. The therapeutically effective dose of drugs administered to adult human patients also depends on the route of administration, the age, weight and condition of the individual. Some patients who fail to respond to one drug may respond to another, and for this reason, several drugs may have to be tried to find the one most effective for an individual patient.

Beyond the specified guidance above, one needs to remember that the dosages depend on the relative potency and bioavailability of the various drugs of choice. These parameters may vary by several fold depending on the drugs being considered. As a preliminary estimate of the dosages in humans, one can look to the rat model and the biological effects provided there as a first guide to dosing in the human with the caveat that one typically dosed the rat with at least 10-fold to 100-fold the amount of the drug to ensure operability under laboratory conditions.

### EXAMPLES

The following examples are provided by way of illustration only and not by way of limitation. Those of skill in the art will readily recognize a variety of non-critical parameters that could be changed or modified to yield essentially similar results.

### Example 1

Chronic recording preparation: Adult male Long-Evans rats with recording and stimulating electrodes chronically implanted into different areas of the brain known to subserve memory were used. The electrodes were stereotaxically inserted under pentobarbital anesthesia (65 mg/kg), using procedures essentially as described in earlier work (Stäubli and Xu, Journal of Neuroscience 15: 2445-2452, 1995; Stäubli and Scafidi, Journal of Neuroscience 17: 4820-4828, 1997). Briefly, monopolar stimulating electrodes (125 µm) were placed into the perforant path projections to hippocampal dentate gyrus and into the lateral olfactory tract projections to olfactory cortex. Following these steps, the leads of the electrodes were connected to a headstage that is permanently affixed to the rat's skull.

The animals were allowed approximately 10 days for recovery before being acclimated to a chronic recording cage (30 x 30 x 58 cm) and the attachment of a recording lead to their headstage. Biphasic stimulation pulses were provided by a digital stimulator that allows precise control of current intensity and pulse duration. Current intensity (10-80 µA) and pulse width (100-150 µsec) were adjusted to produce an evoked field excitatory postsynaptic potential (evoked fEPSP) that is 50-60% of the maximum amplitude of the population spike-free response which typically ranges between 3 and 7 mV. Recording signals were pre-amplified 10x via a PET operational amplifier built into the recording lead and fed into a second stage amplifier set to a gain of 10, with a band-pass of 1 Hz to 5 kHz. The evoked responses were monitored on a storage oscilloscope and digitized at 10 kHz. The peak amplitude, half-width and area of each response were measured on-line and stored on disk for off-line analysis. Baseline evoked EPSPs were tested at 20 see intervals for a minimum of 30 to 45 min, or until stable (<10% variability in amplitude). The animal was free to move around the cage throughout the experiment, but was not allowed to fall asleep.

Drug Testing: Once baseline stability was established over at least 30 min, the animal was given an intraperitoneal injection of 1) Ampakine compound alone (*e.g*., CX717 at 10 mg/kg), or 2) acetylcholinesterase (AChE) inhibitor alone (*e.g*., physostigmine at 0.1 mg/kg, tacrin at 1 mg/kg, galantamine at 1 mg/kg or donezepil at 1.5 mg/kg) or 3) Ampakine compound plus AChE inhibitor combined, both administered at the same time and at the same dose as when given alone. Each rat received each of these three treatments, but only one treatment was given per day, with several days separating successive treatments. The effect of each of these treatments on amplitude size, halfwidth and area of the field EPSP was continuously recorded for several hours after injection.

Results: An ampakine compound at a dose that by itself produced only small facilitatory effects on synaptic transmission (*e.g*., CX717 at 10 mg/kg ip produces an increase in amplitude of 5 + 1 %), combined with a dose of an AChE inhibitor that by itself had no apparent effect on any measures of the field EPSP, caused increases in the size of field EPSPs that were 2- to 6-fold greater than those obtained with the ampakine by itself. The variation in the degree to which the AChE inhibitor enhanced the effect of the ampakine depended on the kind of AChE inhibitor used and the brain area examined. In other words, a class of drugs (AChE inhibitors) that at the dosages used herein produced no effects on monosynaptic responses (field EPSPs) known to be mediated by AMPA receptors, multiplied the facilitatory effects of ampakines on those responses *in vivo.* This effect was obtained in two different synaptic populations at which AMPA receptors are known to mediate the post-synaptic response: the synapses formed by the entorhinal cortex projections to the dentate gyrus of the hippocampus (*i.e*., the perforant path) and the synapses formed by the lateral olfactory tract axons and the olfactory cortex. Figure 1 shows effects of CX717 on responses evoked by stimulation of the lateral olfactory tract (LOT) in the presence and absence of various acetylcholinesterase inhibitors (i.e., Aricept, physostigmine, tacrine). Figure 1A shows that CX717 alone, injected at a dosage of 10 mg/kg, produced minimal increases in the size of LOT EPSPs (∼5%). In contrast, when co-administered with either Aricept (1.5 mg/kg, Figure 1B), Physostigmine (0.1 mg/kg, Figure 1C), or tacrine (1 mg/kg, Figure 1D), CX717 (10 mg/kg) produced increases in response amplitude that were 20-30% above baseline values and several times greater than the effects it produced on its own. Similar effects were obtained with CX1176 (8-acetyl-2,3,8,9-tetrahydro-2,3-pyrrolo-[6,8]oxazino[1,3]benzoxazin-4-one at 1 mg/kg and galantamine at 1 mg/kg (data not shown). When injected alone, the acetylcholinesterase inhibitors produced no measurable effects on LOT responses (Figure 1, E and F). Similar results were observed in the dentate gyrus of freely moving rats (Figure 2). CX717 (10 mg/kg) alone produced minimal (<10%) increases on responses evoked by stimulation of the perforant path fibers (Figure 2A). Yet when CX717 was administered together with an acetylcholinesterase inhibitor (*e.g*., physostigmine at 0.1 mg/kg, as shown in Figure 2B or tacrine at 1 mg/kg, as shown in Figure 2C), perforant path evoked responses were increased by 10-30%. Effects of this kind were also obtained using galantamine (1 mg/kg, data not shown). On their own, acetylcholinesterase inhibitors did not affect the size of perforant path responses at the dosages used herein, as illustrated with physostigmine (0.1 mg/kg) in Figure 2D.

### Example 2

Hippocampal slice preparation: Hippocampal slices were prepared from male Sprague-Dawley rats, approximately 4-6 weeks of age. Rats were anesthetized with halothane and then sacrificed via decapitation. The brain was quickly removed and placed in icy, oxygenated artificial cerebrospinal fluid (ACSF) of the following composition for dissection (in mM): 124 NaCl, 3 KCl, 1.25 KH₂PO₄, 5 MgSO₄, 3.4 CaCl₂, 10 D-glucose, 26 NaHCO₃. A tissue block containing the hippocampus and surrounding tissue was prepared and glued to the stage of a vibrating tissue slicer (Leica VT1000; Bannockburn, IL). Slices were cut roughly perpendicular to the longitudinal axis of the hippocampus at a thickness of 350 µm. Slices were then immediately transferred to an interface recording chamber containing ACSF with the same constituents as described above except that the concentration of MgSO₄ was lowered to 2.5 mM. Recording commenced after a recovery period that was ≥ 1 hour.

*In vitro* recording: Slices in the interface chamber were maintained at 32 ± 1°C and continuously perfused with oxygenated recording ACSF at a rate of 60 ml/br. Additionally, warmed and humidified 95%O₂/5%CO₂ was blown into the chamber from above. The lateral perforant path was stimulated with twisted nichrome wire (65 µm) in the outer molecular layer of the dentate gyrus. A chloride-coated silver wire was placed in a single glass pipette filled with 2 M NaCl (∼5 MΩ resistance) for recording of field potentials. The recording electrode was also placed in the outer molecular layer of the dentate gyrus to sample the perforant path synapses. Stimulation intensity was adjusted to elicit responses that were typically 1-2 mV in amplitude and ≤50% of the maximal monophasic response. Evoked responses were recorded at 10 kHz using a differential AC amplifier (A-M Systems Model 1700; Carlsborg, WA). Baseline responses were monitored every 20 seconds for a minimum of 30 minutes, depending on the length of time necessary to achieve stability.

Drug application: AChE inhibitors enhance transmission at cholinergic synapses by blocking the enzyme that degrades the released transmitter acetylcholine. This causes acetylcholine to remain at relatively high concentrations for a longer period within the synapse and thereby exert a greater than normal effect on acetylcholine receptors. This greater than normal action on acetylcholine receptors can be partially mimicked by applying a compound that directly stimulates the receptors to a degree that is greater than that produced by normally released acetylcholine. Accordingly, for the *in vitro* example presented here, the enhancement of cholinergic transmission produced in vivo by AChE inhibitors was mimicked by using a minimal concentration (0.5 µM) of the cholinergic agonist carbachol (CCh). As noted above, this compound binds to muscarinic type but not nicotinic type acetylcholine receptors. When applied by itself at this low concentration, CCh produced no discernible effects on lateral perforant path EPSPs. This is consistent with the lack of effect produced by AChE inhibitors administered alone at the dosages used in the in vivo example (see above). Additionally, the concentration of ampakine CX717 used in the current example (40 µM) produced very small (<5%) effects on lateral perforant path EPSPs when infused on its own. Each slice tested received one of three treatments: 1) ampakine compound (CX717) alone; 2) CCh alone; or 3) CCh pretreatment followed by infusion of ampakine. The effect of each of these treatments on amplitude size and slope of the descending phase of the response waveform was continuously recorded online throughout the entirety of the experiment.

Results: When the ampakine CX717 was infused at a concentration of 40 µM following a 30-minute pretreatment with 0.5 µM CCh, increases that were significantly greater than those observed in the absence of CCh were observed (Figure 3). Infused on its own for a period of 30 minutes, 40 µM CX717 produced only minor effects on EPSPs (*e.g*., response amplitude was 103.5 ± 1.0% of baseline during the last 10 minutes of drug infusion, Figure 3E). Moreover, 0.5 µM CCh did not detectably affect synaptic transmission when applied by itself (*e.g*., EPSP amplitude was 99.8 ± 2.9% of baseline at 20-30 minutes after the start of infusion, Figure 3D). In contrast, CX717 applied during CCh treatment increased response size significantly (16.6 ± 1.5% above baseline at 20-30 minutes after the start of CX717 infusion, Figure 3A-B). Thus, CX717 infused in the presence of CCh produced increases in response size that were several fold greater than those produced by CX717 alone. These results confirm the conclusion from the *in vivo* experiments that stimulation of cholinergic receptors enhances the potency of AMPA receptor potentiators.

### Example 3

The following example provides guidance for use in humans. A 60-year old male who weighs 70 kg and suffers from Alzheimer's Disease is provided with donezepil at a dosage of 5-10 mg per day in combination with CX717 at a dosage of 10-100 mg per day. Therapy is continued and cognitive improvement is assessed by any of the standard psychological tests such as the Folstein mini-mental examination.

To determine whether a specific combination of AChE inhibitor and AMPA receptor potentiator works at the concentrations provided, one first prepares adult rats for chronic recording of evoked field EPSPs in the hippocampus or cortex. Next, the AMPA receptor potentiator, such as an ampakine, is administered at doses that produce small, but reliable, synaptic facilitation of EPSP amplitude (between 5-10% in hippocampus or other brain structures). Next, one can test the same dose of AMPA receptor potentiator together with any AChE inhibitor at a dose described in the literature as effective at enhancing acetylcholine mediated transmission; *e.g*., a dose that reduces the behavioral effects of drugs that block acetylcholine receptors in rats (*e.g*., physostigmine at 0.1 mg/kg, donezepil at 1.5 mg/kg). AChE inhibitors by themselves do not increase monosynaptic EPSPs mediated by AMPA receptors. Thus, one can then determine the degree of synaptic facilitation observed with the combined treatment relative to that obtained with AMPA receptor potentiator alone. Any statistically meaningful increase in the monosynaptic field EPSP is an indication of a facilitating effect by the AChE inhibitor.

## Claims

1. A composition comprising an effective amount of an AMPA receptor potentiator and an effective amount of an acetylcholinesterase inhibitor for use in therapy in an animal, wherein the therapeutic or physiological effect of the AMPA receptor potentiator is enhanced.

2. A composition comprising an effective amount of an AMPA receptor potentiator, an effective amount of an acetylcholinesterase inhibitor, and a physiologically acceptable carrier.

3. The composition of claim 1 or 2, wherein the potentiator is an AMPAkine.

4. The composition of claim 1 or 2, wherein the acetylcholinesterase inhibitor is donepezil hydrochloride.

5. The composition of claim 1 or 2, which is formulated for peripheral administration.

6. The composition of claim 5, wherein the peripheral administration is oral administration.

7. The composition of claim 1, wherein the animal is a human.

8. Use of an effective amount of an AMPA receptor potentiator and an effective amount of an acetylcholinesterase inhibitor in the manufacture of a medicament for treating cognitive disorder in an animal.

9. The use of claim 8, wherein the potentiator is an AMPAkine.

10. The use of claim 8, wherein the acetylcholinesterase inhibitor is donepezil hydrochloride.

11. The use of claim 8, wherein the cognitive disorder is selected from the group comprising Alzheimer's Disease, senile dementure, attention deficit disorder (ADD), mild cognitive impairment(MCI), schizophrenia, depression, sexual dysfunctional, anxiety, and impaired performance after sleep deprivation.

12. The use of claim 8, wherein the animal is a human.

13. The use of claim 8, wherein the administering is by peripheral administration.

## Patentansprüche

1. Zusammensetzung umfassend eine wirksame Menge eines AMPA-Rezeptor-Verstärkers und eine wirksame Menge eines Acetylcholinesterase-Inhibitors zur Verwendung in der Therapie in einem Tier, wobei die therapeutische oder physiologische Wirkung des AMPA-Rezeptor-Verstärkers verstärkt wird.

2. Zusammensetzung umfassend eine wirksame Menge eines AMPA-Rezeptor-Verstärkers, eine wirksame Menge eines Acetylcholinesterase-Inhibitors und einen physiologisch verträglichen Träger.

3. Zusammensetzung nach Anspruch 1 oder 2, wobei der Verstärker ein AMPAkin ist.

4. Zusammensetzung nach Anspruch 1 oder 2, wobei der Acetylcholinesterase-Inhibitor Donepezil hydrochlorid ist.

5. Zusammensetzung nach Anspruch 1 oder 2, welche für die periphere Verabreichung formuliert ist.

6. Zusammensetzung nach Anspruch 5, wobei die periphere Verabreichung orale Verabreichung ist.

7. Zusammensetzung nach Anspruch 1, wobei das Tier ein Mensch ist.

8. Verwendung einer wirksamen Menge eines AMPA-Rezeptor-Verstärkers und einer wirksamen Menge eines Acetylcholinesterase-Inhibitors zur Herstellung eines Medikaments zur Behandlung einer kognitiven Störung in einem Tier.

9. Verwendung nach Anspruch 8, der Verstärker ein AMPAkin ist.

10. Verwendung nach Anspruch 8, wobei der Acetylcholinesterase-Inhibitor Donepezil hydrochlorid ist.

11. Verwendung nach Anspruch 8, wobei die kognitive Störung aus der Gruppe, umfassend Alzheimer-Krankheit, senile Demenz, Aufmerksamkeitsdefizitsyndrom (ADS), leichte kognitive Beeinträchtigung (MCI), Schizophrenie, Depression, sexuelle Dysfunktion, Angst und Leistungsstörung nach Schlafentzug, ausgewählt ist.

12. Verwendung nach Anspruch 8, wobei das Tier ein Mensch ist.

13. Verwendung nach Anspruch 8, wobei die Verabreichung mittels peripherer Verabreichung ist.

## Revendications

1. Composition comprenant une quantité efficace d'un potentialisateur des récepteurs d'AMPA et une quantité efficace d'un inhibiteur d'acétylcholinestérase pour une utilisation en thérapie chez un animal, l'effet thérapeutique ou physiologique du potentialisateur des récepteurs d'AMPA étant augmenté.

2. Composition comprenant une quantité efficace d'un potentialisateur des récepteurs d'AMPA, une quantité efficace d'un inhibiteur d'acétylcholinestérase et un support physiologiquement acceptable.

3. Composition suivant la revendication 1 ou 2, dans laquelle le potentialisateur est l'AMPAkine.

4. Composition suivant la revendication 1 ou 2, dans laquelle l'inhibiteur d'acétylcholinestérase est le chlorhydrate de donépézil.

5. Composition suivant la revendication 1 ou 2, qui est formulée pour l'administration périphérique.

6. Composition suivant la revendication 5, l'administration périphérique étant l'administration orale.

7. Composition suivant la revendication 1, l'animal étant un être humain.

8. Utilisation d'une quantité efficace d'un potantialisateur des récepteurs d'AMPA et d'une quantité efficace d'un inhibiteur d'acétylcholinestérase dans la production d'un médicament pour le traitement d'un trouble cognitif chez un animal.

9. Utilisation suivant la revendication 8, dans laquelle le potentialisateur est une AMPAkine.

10. Utilisation suivant la revendication 8, dans laquelle l'inhibiteur d'acétylcholinestérase est le chlorhydrate de donépézil.

11. Utilisation suivant la revendication 8, dans laquelle le trouble cognitif est choisi dans le groupe comprenant la maladie d'Alzheimer, la démence sénile, le trouble de déficit d'attention (ADD), l'altération cognitive bénigne (MCI), la schizophrénie, la dépression, le dysfonctionnement sexuel, l'anxiété et une altération des performances après une privation de sommeil.

12. Utilisation suivant la revendication 8, dans laquelle l'animal est un être humain.

13. Utilisation suivant la revendication 8, dans laquelle l'administration est effectuée par administration périphérique.
